# EUROPEAN PATENT APPLICATION

(11) **EP 4 512 794 A1**
(43) Date of publication of application: **26.02.2025**
(21) Application number: 23791837.0
(22) Date of filing: 17.04.2023
(51) Int. Cl.: C07C 41/44, C07C 43/12

(54) **METHOD FOR PRODUCING COMPOSITION CONTAINING PURIFIED FLUORINE-CONTAINING ETHER COMPOUND**

(30) Priority: 20.04.2022 JP 2022069500
(71) Applicant: DAIKIN INDUSTRIES, LTD., Osaka-shi, Osaka 530-0001 (JP)
(72) Inventor: OKADA, Michiaki, Osaka-Shi, Osaka 530-0001 (JP); YOSHIYAMA, Asako, Osaka-Shi, Osaka 530-0001 (JP); KISHIMOTO, Masayuki, Osaka-Shi, Osaka 530-0001 (JP); KAWAKAMI, Yuka, Osaka-Shi, Osaka 530-0001 (JP); KUROKI, Yoshichika, Osaka-Shi, Osaka 530-0001 (JP); HOSHIYA, Naoyuki, Osaka-Shi, Osaka 530-0001 (JP); MARI, Daichi, Osaka-Shi, Osaka 530-0001 (JP)
(74) Representative: Hoffmann Eitle
(86) International application number: PCT/JP2023/015368
(87) International publication number: WO 2023/204189

(57) **Abstract**

There is provided a method for producing a composition containing a purified fluorine-containing ether compound.

A method for producing a composition containing a purified fluorine-containing ether compound, wherein
the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time, and
the method includes:
(A) converting, through a reaction between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound in the presence of the fluorine-containing ether compound represented by general formula (1), the fluorine-containing olefin compound represented by general formula (2) into an adduct of the nitrogen-containing compound and/or a fluorine-containing carboxylic acid analogue to obtain a composition containing the fluorine-containing ether compound represented by general formula (1) and the adduct of the nitrogen-containing compound and/or the fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound; and
(B) separating the adduct of the nitrogen-containing compound and/or the fluorine-containing carboxylic acid analogue from the composition obtained in step (A) to obtain the composition containing a purified fluorine-containing ether compound.

## Description

### Technical Field

The present disclosure relates to a method for producing a composition containing a purified fluorine-containing ether compound.

### Background Art

In recent years, hydrofluoroethers (HFEs) have attracted attention as a substitute for chlorofluorocarbons (CFCs) and hydrochlorofluorocarbons (HCFCs) due to their low GWP (Global Warming Potential) and ODP (Ozone Depletion Potential), and low toxicity.

PTL 1 discloses that a primary amine and/or a secondary amine are allowed to act on a composition containing a fluorine-containing ether and a fluorine-containing olefin, thereby converting the fluorine-containing olefin into a high-boiling point compound, and subsequent distillation and purification yields a purified fluorine-containing ether.

### Citation List

### Patent Literature

PTL 1: JP 2008-230981 A

### Summary of Invention

### Technical Problem

An object of the present disclosure is to provide a method for producing a composition containing a purified fluorine-containing ether compound represented by general formula (1): CHX¹X²CF₂OX^{3,} wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time.

### Solution to Problem

The present disclosure encompasses the inventions described in the following items.
Item 1. A method for producing a composition containing a purified fluorine-containing ether compound, wherein
   the fluorine-containing ether compound is represented by general formula (1): CHX¹X²CF₂OX^{3,} wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time, and
   the method includes:
   (A) converting, through a reaction between a fluorine-containing olefin compound represented by general formula (2): CX¹X²=CF (CF₂) ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound in the presence of the fluorine-containing ether compound represented by general formula (1), the fluorine-containing olefin compound represented by general formula (2) into an adduct of the nitrogen-containing compound and/or a fluorine-containing carboxylic acid analogue to obtain a composition containing the fluorine-containing ether compound represented by general formula (1) and the adduct of the nitrogen-containing compound and/or the fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound; and
   (B) separating the adduct of the nitrogen-containing compound and/or the fluorine-containing carboxylic acid analogue from the composition obtained in step (A) to obtain the composition containing a purified fluorine-containing ether compound.
Item 2. The production method according to item 1, wherein the nitrogen-containing compound is at least one selected from the group consisting of an amide compound, a urea compound, a urethane compound, an imide compound, and a tertiary amine compound.
Item 3. The production method according to item 1 or 2, wherein in step (B), the adduct of the nitrogen-containing compound and/or the fluorine-containing carboxylic acid analogue are separated from the composition obtained in step (A) by distillation of the composition obtained in step (A).
Item 4. The production method according to any one of items 1 to 3, wherein the fluorine-containing ether compound represented by general formula (1) is 1,1,2,3,3,3-hexafluoropropyl methyl ether (HFE-356mec).
Item 5. The production method according to any one of items 1 to 4, wherein the fluorine-containing olefin compound represented by general formula (2) is CF₃CF=CFOCH₃ and/or CF₂=CFCF₂OCH₃.
Item 6. A composition containing a fluorine-containing ether compound, wherein
   the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
   the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
   a total content of the adduct of the nitrogen-containing compound and/or the fluorine-containing carboxylic acid analogue is 5% by mass or less with respect to a mass of the fluorine-containing ether compound.
Item 7. A composition containing a fluorine-containing ether compound, wherein
   the fluorine-containing ether compound is represented by general formula (1): CHX¹X²CF₂OX^{3,} wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
   the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
   a content of the adduct of the nitrogen-containing compound is 3% by mass or less with respect to a mass of the fluorine-containing ether compound, and a content of the fluorine-containing carboxylic acid analogue is 2% by mass or less with respect to a mass of the fluorine-containing ether compound.
Item 8. A composition containing a fluorine-containing ether compound, wherein
   the fluorine-containing ether compound is 1,1,2,3,3,3-hexafluoropropyl methyl ether (HFE-356mec),
   the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
   a total content of the adduct of the nitrogen-containing compound and/or the fluorine-containing carboxylic acid analogue is 5% by mass or less with respect to a mass of the fluorine-containing ether compound.
Item 9. A composition containing a fluorine-containing ether compound, wherein
   the fluorine-containing ether compound is 1,1,2,3,3,3-hexafluoropropyl methyl ether (HFE-356mec),
   the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
   a content of the adduct of the nitrogen-containing compound is 3% by mass or less with respect to a mass of the fluorine-containing ether compound, and a content of the fluorine-containing carboxylic acid analogue is 2% by mass or less with respect to a mass of the fluorine-containing olefin compound.
Item 10. A composition containing a fluorine-containing ether compound, wherein
   the fluorine-containing ether compound is represented by general formula (1): CHX¹X²CF₂OX^{3,} wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
   the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
   a total content of the adduct of the nitrogen-containing compound and/or the fluorine-containing carboxylic acid analogue is 0.2% by mass or less with respect to a mass of the fluorine-containing ether compound.
Item 11. A composition containing a fluorine-containing ether compound, wherein
   the fluorine-containing ether compound is represented by general formula (1): CHX¹X²CF₂OX^{3,} wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
   the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
   a content of the adduct of the nitrogen-containing compound is 0.1% by mass or less with respect to a mass of the fluorine-containing ether compound, and a content of the fluorine-containing carboxylic acid analogue is 0.1% by mass or less with respect to a mass of the fluorine-containing ether compound.
Item 12. A composition containing a fluorine-containing ether compound, wherein
   the fluorine-containing ether compound is 1,1,2,3,3,3-hexafluoropropyl methyl ether (HFE-356mec),
   the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
   a total content of the adduct of the nitrogen-containing compound and/or the fluorine-containing carboxylic acid analogue is 0.2% by mass or less with respect to a mass of the fluorine-containing ether compound.
Item 13. A composition containing a fluorine-containing ether compound, wherein
   the fluorine-containing ether compound is 1,1,2,3,3,3-hexafluoropropyl methyl ether (HFE-356mec),
   the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
   a content of the adduct of the nitrogen-containing compound is 0.1% by mass or less with respect to a mass of the fluorine-containing ether compound, and a content of the fluorine-containing carboxylic acid analogue is 0.1% by mass or less with respect to a mass of the fluorine-containing ether compound.
Item 14. A method for producing a composition containing a purified fluorine-containing ether compound, wherein
   the fluorine-containing ether compound is represented by general formula (1): CHX¹X²CF₂OX^{3,} wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time, and
   the method includes:
   (A) converting, through a reaction between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound with a Mulliken charge density value on a nitrogen atom of -0.65 or more and 0.1 or less in the presence of the fluorine-containing ether compound represented by general formula (1), the fluorine-containing olefin compound represented by general formula (2) into an adduct of the nitrogen-containing compound and/or a fluorine-containing carboxylic acid analogue to obtain a composition containing the fluorine-containing ether compound represented by general formula (1) and the adduct of the nitrogen-containing compound and/or the fluorine-containing carboxylic acid analogue; and
   (B) separating the adduct of the nitrogen-containing compound and/or the fluorine-containing carboxylic acid analogue from the composition obtained in step (A) to obtain the composition containing a purified fluorine-containing ether compound.
Item 15. A composition containing a fluorine-containing ether compound, wherein
   the fluorine-containing ether compound is represented by general formula (1): CHX¹X²CF₂OX^{3,} wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
   the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound with a Mulliken charge density value on a nitrogen atom of -0.65 or more and 0.1 or less, the reaction product being an adduct of the nitrogen-containing compound and/or a fluorine-containing carboxylic acid analogue, and
   a total content of the adduct of the nitrogen-containing compound and/or the fluorine-containing carboxylic acid analogue is 0.2% by mass or less with respect to a mass of the fluorine-containing ether compound.

### Advantageous Effects of Invention

According to the production method of the present disclosure, a composition containing a purified fluorine-containing ether compound represented by general formula (1) described above can be produced by a simple and convenient method.

### Description of Embodiments

As a result of diligent studies, the present disclosers have found that the object described above can be achieved by utilizing a reaction between a fluorine-containing olefin compound represented by general formula (2) described above and a specific nitrogen-containing compound.

The present disclosure was completed as a result of further studies based on such a finding. Hereinafter, embodiments included in the present disclosure will be described in detail.

In the present specification, the expressions "containing" and "including" include the concepts of "containing", "including", "consisting substantially of", and "consisting solely of".

In the present specification, "purity" means the component ratio (% by mass) as determined by quantitative analysis by gas chromatography (GC).

In the present specification, "reflux ratio" means the molar flow rate ratio between reflux and distillate (reflux/distillate).

In the present specification, "gauge pressure" refers to the relative pressure based on atmospheric pressure, and means the pressure difference obtained by subtracting the atmospheric pressure from the absolute pressure. In the present specification, gauge pressure is denoted with "G" as in MPaG, for example. On the other hand, cases where "G" is not added indicate absolute pressure.

In the present specification, "A and/or B" means either A or B, or both A and B.

In the present specification, "alkyl group having 1 to 6 carbon atoms" means a methyl group, an ethyl group, a propyl group, an isopropyl group, a butyl group, a sec-butyl group, a tert-butyl group, a pentyl group, and a hexyl group.

### 1. Method for Producing Composition Containing Purified Fluorine-Containing Ether Compound

The method for producing a composition containing a purified fluorine-containing ether compound of the present disclosure (hereinafter, also simply referred to as "production method of the present disclosure") includes the following step (A) and step (B) in this order. Hereinafter, the production method of the present disclosure will be described in the order of steps (A) and (B).

### Step (A)

Step (A) is a step of converting, in the presence of a fluorine-containing ether compound represented by general formula (1): CHX¹X²CF₂OX^{3,} wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time, through a reaction between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the fluorine-containing olefin compound represented by general formula (2) into an adduct of the nitrogen-containing compound and/or a fluorine-containing carboxylic acid analogue to obtain a composition containing the fluorine-containing ether compound represented by general formula (1) and the adduct of the nitrogen-containing compound and/or the fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound.

In the present disclosure, the fluorine-containing ether compound is represented by general formula (1) : CHX¹X²CF₂OX^{3,} wherein X¹, X², and X³ are the same as described above. Specific examples thereof include CF₃CHFCF₂OX³, CHF₂CF₂OX³, CH₂FCF₂OX³, and CH₃CF₂OX³ (in all cases, X³ represents an alkyl group having 1 to 6 carbon atoms and optionally containing a fluorine atom).

The fluorine-containing ether compound represented by general formula (1) is preferably at least one compound selected from the group consisting of CF₃CHFCF₂OCH₃, CHF₂CF₂OCH₃, CH₂FCF₂OCH₃, and CH₃CF₂OCH₃. Among these, CF₃CHFCF₂OCH₃ (1,1,2,3,3,3-hexafluoropropyl methyl ether: HFE-356mec) is more preferred.

In the present disclosure, the fluorine-containing olefin compound is represented by general formula (2): CX¹X²=CF (CF₂) ₙOX³, wherein X¹, X², X³, and n are the same as described above. Specific examples thereof include CF₃CF=CFOX³, CF₂=CFCF₂OX³, CF₂=CFOX³, CHF=CFOX³, and CH₂=CFOX³ (in all cases, X³ represents an alkyl group having 1 to 6 carbon atoms and optionally containing a fluorine atom).

The fluorine-containing olefin compound represented by general formula (2) is preferably at least one compound selected from the group consisting of CF₃CF=CFOCH₃, CF₂=CFCF₂OCH₃, CF₂=CFOCH₃, CHF=CFOCH₃, and CH₂=CFOCH₃. Among these, at least one of CF₃CF=CFOCH₃ and CF₂=CFCF₂OCH₃ is more preferred.

In the present disclosure, as the nitrogen-containing compound that is allowed to react with the fluorine-containing olefin compound, nitrogen-containing compound A having a structure in which an acyl group is bonded to a nitrogen atom and/or nitrogen-containing compound B that is a tertiary amine compound are used. Hereinafter, unless otherwise noted, the term nitrogen-containing compound is a generic term for "nitrogen-containing compound A and/or nitrogen-containing compound B".

Examples of nitrogen-containing compound A having a structure in which an acyl group is bonded to a nitrogen atom (hereinafter, also simply referred to as "nitrogen-containing compound A") include at least one selected from the group consisting of an amide compound, a urea compound, a urethane compound, and an imide compound having such a structure. In the case where these compounds are used in combination, examples of the combination include a mixture of an amide compound and a urea compound, a mixture of an amide compound and a urethane compound, a mixture of an amide compound and an imide compound, a mixture of a urea compound and a urethane compound, a mixture of a urea compound and an imide compound, and a mixture of a urethane compound and an imide compound.

Examples of each compound include, in detail, at least one selected from the group consisting of a chain amide compound represented by general formula (3), a cyclic amide compound represented by general formula (4), a chain urea compound represented by general formula (5), a cyclic urea compound represented by general formula (6), a chain urethane compound represented by general formula (7), a cyclic urethane compound represented by general formula (8), a chain imide compound represented by general formula (9), and a cyclic imide compound represented by general formula (10). 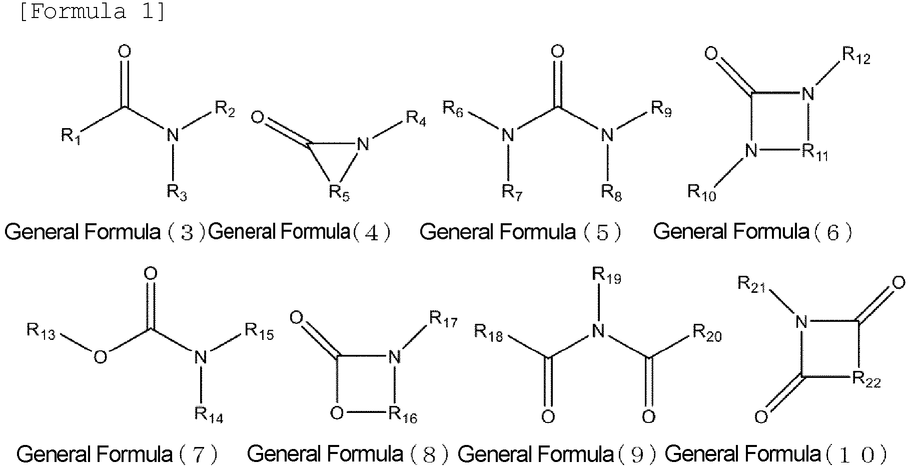 In the formulae, R₁, R₂, R₃, R₄, R₆, R₇, R₈, R₉, R₁₀, R₁₂, R₁₄, R₁₅, R₁₇, R₁₉, and R₂₁ are the same or different and represent a hydrogen atom or an alkyl group having 1 to 6 carbon atoms and optionally having a substituent (of this, a hydrogen atom or an alkyl group having 1 to 3 carbon atoms and optionally having a substituent is preferred.). R₅ represents an alkyl group having 1 to 6 carbon atoms and optionally having a substituent (of this, an alkyl group having 1 to 3 carbon atoms and optionally having a substituent is preferred. ) . R₁₁, R₁₃, R₁₆, R₁₈, R₂₀, and R₂₂ represent an alkyl group having 1 to 6 carbon atoms and optionally having a substituent (of this, an alkyl group having 1 to 3 carbon atoms and optionally having a substituent is preferred.).

Specific examples of the amide compound include formamide, acetamide, N,N-dimethylformamide (DMF), N,N-dimethylacetamide (DMAc), diethylacetamide, dipropylacetamide, dibutylacetamide, dimethylisobutylamide, dimethylpropylamide, 3-methoxy-N,N-dimethylpropionamide, N-methylpyrrolidone (NMP), N-ethylpyrrolidone, 2-pyrrolidone, N-methylpiperidone, benzamide, and N-phenylacetamide, which can be used without being limited to a chain structure or a cyclic structure. Among these amide compounds, for reasons of ease of availability, N,N-dimethylformamide (DMF) or N,N-dimethylacetamide (DMAc) is preferred, and N,N-dimethylformamide (DMF) is more preferred. These amide compounds may be used alone, or in combination of two or more.

Examples of the urea compound include urea, 1,3-dimethylurea, tetramethylurea, 1,3-diethylurea, tetraethylurea, dipropylurea, tetrapropylurea, dibutylurea, tetrabutylurea, 1,3-dimethyl-2-imidazolidinone (DMI), 2-imidazolidinone, tetrahydro-2-pyrimididone, diphenylurea, and phenylurea, which can be used without being limited to a chain structure or a cyclic structure. Among these urea compounds, for reasons of ease of availability, tetramethylurea is preferred. These urea compounds may be used alone, or in combination of two or more.

Examples of the urethane compound include 2-azetidinone, 2-pyrrolidone, ethyl N-methylcarbamate, 2-oxazolidone, 3-methyl-2-oxazolidone, methyl N-phenylcarbamate, phenylurethane, methyl N-methylcarbamate, methyl carbamate, ethyl N-ethylcarbamate, ethyl carbamate, propyl N-propylcarbamate, propyl carbamate, butyl N-butylcarbamate, butyl carbamate, and phenyl carbamate, which can be used without being limited to a chain structure or a cyclic structure. Among these urethane compounds, for reasons of ease of availability, 2-pyrrolidone and 2-oxazolidone are preferred, and 2-oxazolidone is more preferred. These urethane compounds may be used alone, or in combination of two or more.

Examples of the imide compound include N-methyldiacetamide, diacetamide, N-methylsuccinimide, N-methylmaleimide, N-ethylsuccinimide, N-ethylmaleimide, N-acetyl-γ-caprolactam, N-methylphthalimide, N-phenylmaleimide, N-phenylsuccinimide, N-methylbis(trifluoroacetamide), N-methyl-N-(1-oxopropyl)propanamide, N-ethyl-N-(1-oxopropyl)propanamide, N-butyl-N-(1-oxopropyl)butanamide, N-(1-oxopropyl)propanamide, and N-(1-oxopropyl)butanamide, which can be used without being limited to a chain structure or a cyclic structure. Among these imide compounds, for reasons of ease of availability, N-methyldiacetamide is preferred. These imide compounds may be used alone, or in combination of two or more.

Examples of nitrogen-containing compound B that is a tertiary amine compound (hereinafter, also simply referred to as "nitrogen-containing compound B") include a chain amine compound, a cyclic amine compound, and a heterocyclic aromatic compound, which can be used without being limited to a chain structure or a cyclic structure. Specific examples of the tertiary amine compound include trimethylamine, triethylamine, tributylamine, diazabicycloundecene, N-methylpyrrolidine, pyridine, 4-dimethylaminopyridine, and N,N-dimethylbenzylamine. Among these amine compounds, for reasons of ease of availability, triethylamine, diazabicycloundecene, and 4-dimethylaminopyridine are preferred. Nitrogen-containing compound B that is a tertiary amine compound may be used alone, or in combination of two or more.

In the present disclosure, the nitrogen-containing compound is at least one selected from the group consisting of an amide compound, a urea compound, a urethane compound, an imide compound, and a tertiary amine compound. Specifically, the nitrogen-containing compound is preferably at least one selected from the group consisting of N,N-dimethylformamide (DMF), N,N-dimethylacetamide (DMAc), 3-methoxy-N,N-dimethylpropionamide, N-methylpyrrolidone (NMP), urea, tetramethylurea, 1,3-dimethyl-2-imidazolidinone (DMI), 2-azetidinone, 2-pyrrolidone, ethyl N-methylcarbamate, 3-methyl-2-oxazolidone, 2-oxazolidone, N-methyldiacetamide, N-methylsuccinimide, trimethylamine, triethylamine, diazabicycloundecene, N-methylpyrrolidine, pyridine, 4-dimethylaminopyridine, and N,N-dimethylbenzylamine.

When identified by the Mulliken charge density on a nitrogen atom, the nitrogen-containing compound that can be used in the present disclosure can be identified as a nitrogen-containing compound with a Mulliken charge density on a nitrogen atom of -0.65 or more and 0.1 or less. The Mulliken charge density on a nitrogen atom only needs to be -0.65 or more and 0.1 or less, but in the case of nitrogen-containing compound A described above, the Mulliken charge density on a nitrogen atom is preferably -0.65 or more and 0 or less, of which -0.50 or more and 0 or less is more preferred and -0.40 or more and 0 or less is still more preferred. Also, in the case of nitrogen-containing compound B described above, the Mulliken charge density on a nitrogen atom is preferably -0.50 or more and 0.1 or less, of which -0.50 or more and 0.1 or less is more preferred and -0.40 or more and 0.1 or less is still more preferred. Note that the Mulliken charge density in the present disclosure is the charge density obtained by performing structural optimization calculation using quantum chemical calculation software Gaussian16 from Gaussian, Inc. by the density functional theory (DFT) with B3LYP as the functional and 6-31G+(d) as the basis function.

As described above, when identified by the Mulliken charge density on a nitrogen atom, the nitrogen-containing compound that can be used in the present disclosure can be identified as a nitrogen-containing compound with a Mulliken charge density on a nitrogen atom of -0.65 or more and 0.1 or less, and therefore, the method for producing a composition containing a purified fluorine-containing ether compound of the present disclosure can also be described as follows:
"A method for producing a composition containing a purified fluorine-containing ether compound, wherein
the fluorine-containing ether compound is represented by general formula (1): CHX¹X²CF₂OX^{3,} wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time, and
the method includes:
(A) converting, through a reaction between a fluorine-containing olefin compound represented by general formula (2): CX¹X²=CF (CF₂) ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound with a Mulliken charge density value on a nitrogen atom of -0.65 or more and 0.1 or less in the presence of the fluorine-containing ether compound represented by general formula (1), the fluorine-containing olefin compound represented by general formula (2) into an adduct of the nitrogen-containing compound and/or a fluorine-containing carboxylic acid analogue to obtain a composition containing the fluorine-containing ether compound represented by general formula (1) and the adduct of the nitrogen-containing compound and/or the fluorine-containing carboxylic acid analogue; and
(B) separating the adduct of the nitrogen-containing compound and/or the fluorine-containing carboxylic acid analogue from the composition obtained in step (A) to obtain the composition containing a purified fluorine-containing ether compound.".

In step (A), in the presence of the fluorine-containing ether compound represented by general formula (1), the fluorine-containing olefin compound represented by general formula (2) and the nitrogen-containing compound are allowed to react with each other. Through the reaction between the fluorine-containing olefin compound represented by general formula (2) and the nitrogen-containing compound, an adduct of the nitrogen-containing compound and/or a carboxylic acid analogue of the fluorine-containing olefin compound represented by general formula (2) can be obtained as a reaction product. As a result, in step (A), a composition containing the fluorine-containing ether compound represented by general formula (1) and the adduct of the nitrogen-containing compound and/or the carboxylic acid analogue can be obtained.

Among the adduct of the nitrogen-containing compound, the adduct in the case where nitrogen-containing compound A is used as the nitrogen-containing compound is represented by general formulae (11) to (13). 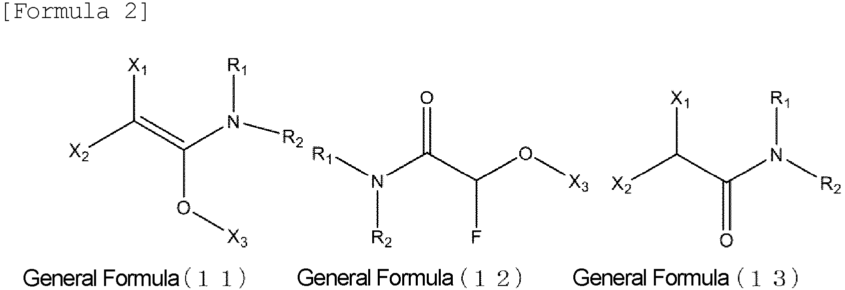 In the formulae, X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time. R₁ and R₂ are the same or different and represent a hydrogen atom or an alkyl group having 1 to 6 carbon atoms and optionally having a substituent (of this, a hydrogen atom or an alkyl group having 1 to 3 carbon atoms and optionally having a substituent is preferred.).

For example, in the case where the fluorine-containing olefin compound represented by general formula (2) is CF₃CF=CFOCH₃ and the nitrogen-containing compound is dimethylformamide (DMF), CF₃CHF-C(=O)-N(CH₃)₂ can be obtained as the adduct. Also, in the case where the fluorine-containing olefin compound represented by general formula (2) is CF₂=CFCF₂OCH₃ and the nitrogen-containing compound is dimethylformamide (DMF), N(CH₃)₂-C(=O)-CFCF₂OCH₃ can be obtained as the adduct.

Also, among the fluorine-containing carboxylic acid analogue, the fluorine-containing carboxylic acid analogue in the case where nitrogen-containing compound A is used as the nitrogen-containing compound is represented by general formulae (14) to (15). 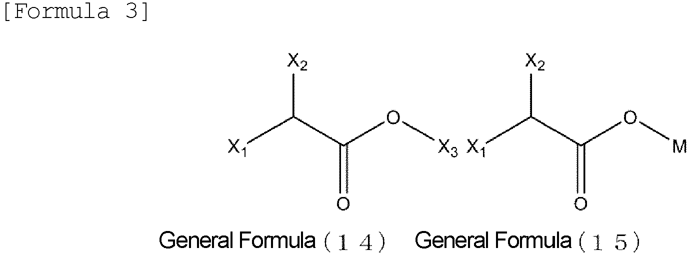 In the formulae, X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time. M represents Li, Na, or K.

For example, in the case where the fluorine-containing olefin compound represented by general formula (2) is CF₃CF=CFOCH₃ and the nitrogen-containing compound is N,N-dimethylformamide (DMF), CF₃CFHCOOMe (Me = methyl group) and/or CF₃CFHCOOM (M = Li, Na, or K) can be obtained as the carboxylic acid analogue.

Also, the reaction product with the fluorine-containing olefin compound in the case where nitrogen-containing compound B is used as the nitrogen-containing compound is mainly a fluorine-containing carboxylic acid analogue, and the fluorine-containing carboxylic acid analogues represented by general formulae (14) to (15) can be obtained.

The content proportion of the fluorine-containing olefin compound represented by general formula (2) prior to the reaction in step (A) is preferably 5% by mass or less with respect to the fluorine-containing ether compound represented by general formula (1).

The reaction in step (A) is preferably a contact reaction between the fluorine-containing olefin compound represented by general formula (2) and the nitrogen-containing compound.

The amount of the nitrogen-containing compound supplied is preferably 1 mole or more, still more preferably 2 moles or more, and preferably 50 moles or less, still more preferably 40 moles or less, with respect to 1 mole of the fluorine-containing olefin compound represented by general formula (2).

In the reaction of step (A), an inert gas component such as nitrogen gas may be coexistent from the viewpoint of suppressing heat generation.

In the reaction of step (A), an inorganic base such as sodium hydroxide and potassium hydroxide may be coexistent from the viewpoint of reducing the amount of the nitrogen-containing compound used. Also, since water and an inorganic base may be coexistent in step (A), step (A) can be performed continuously by adding the nitrogen-containing compound to the composition after performing the reaction shown by reaction formula (X) described below.

Reaction formula (X) shows that, through a reaction between a fluorine-containing olefin and an alcohol in the presence of water and an inorganic base, a fluorine-containing ether compound and a fluorine-containing olefin compound can be obtained, and the product fluorine-containing ether compound corresponds to the fluorine-containing ether compound represented by general formula (1) in the present disclosure, and the product fluorine-containing olefin compound corresponds to the fluorine-containing olefin compound represented by general formula (2) in the present disclosure. Thus, by adding the nitrogen-containing compound to the composition after performing reaction formula (X), step (A) of the present disclosure can be performed in succession to reaction formula (X). Also, since the fluorine-containing carboxylic acid analogue produced by the reaction between the fluorine-containing olefin compound, which is a product of reaction formula (X), and the nitrogen-containing compound is selectively a water-soluble fluorine-containing carboxylic acid salt, the purification load in the subsequent step (B) can also be reduced. 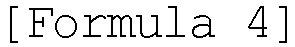 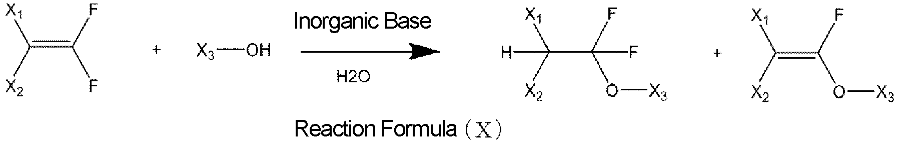 In the formula, X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time.

Examples of the inorganic base that may be coexistent include sodium hydroxide, potassium hydroxide, lithium hydroxide, calcium hydroxide, sodium bicarbonate, potassium bicarbonate, sodium carbonate, potassium carbonate, lithium carbonate, lithium phosphate, sodium phosphate, and potassium phosphate.

The equivalent amount of the inorganic base used is preferably 0.1 equivalents or more, and more preferably 0.2 equivalents or more, with respect to the fluorine-containing ether compound represented by general formula (1). Also, it is preferably 1 equivalent or less, more preferably 0.75 equivalents or less, and still more preferably 0.5 equivalents or less.

The reaction in step (A) can be performed in liquid phase or gas phase. In the case where the reaction is performed in liquid phase, there is a method in which the nitrogen-containing compound is introduced into a crude solution containing the fluorine-containing ether compound represented by general formula (1) and the fluorine-containing olefin compound represented by general formula (2) to bring the crude solution and the nitrogen-containing compound into contact with each other in a reactor. In the case where the reaction is performed in gas phase, there is a method in which a gasified crude solution and the nitrogen-containing compound are brought into contact with each other in a reactor. From the viewpoint of improving the recovery rate of the fluorine-containing ether compound represented by general formula (1), the reaction in step (A) is preferably performed in liquid phase.

In the case where the reaction in step (A) is performed in liquid phase, the reaction temperature is preferably 0°C or higher, and preferably 100°C or lower, more preferably 60°C or lower. Also, the reaction pressure is preferably 0.0 MPaG or more, and preferably 0.5 MPaG or less, more preferably under atmospheric pressure. Furthermore, the reaction time is preferably 0.1 hours or longer, and preferably 24 hours or shorter.

In the case where the reaction in step (A) is performed in gas phase, it is preferably performed in the presence of a catalyst. Examples of the catalyst include activated carbon, zeolite, alumina, and silica alumina.

In the case where the reaction in step (A) is performed in gas phase, the reaction temperature is preferably 70°C or higher, and preferably 300°C or lower. Also, the reaction pressure is preferably -0.05 MPaG or more, and preferably 1.00 MPaG or less. Furthermore, the reaction time is preferably 0.1 hours or longer, and preferably 24 hours or shorter.

Examples of the reactor used in the liquid phase reaction in step (A) include a glass container, a glass-lined container, a resin-lined container, and a SUS container. Also, examples of the reactor used in the gas phase reaction in step (A) include a glass container, a glass-lined container, a resin-lined container, and a SUS container.

### Step (B)

Step (B) is a step of separating the adduct of the nitrogen-containing compound and/or the fluorine-containing carboxylic acid analogue from the composition obtained in step (A) containing the fluorine-containing ether compound represented by general formula (1) and the adduct of the nitrogen-containing compound and/or the fluorine-containing carboxylic acid analogue to obtain a composition containing a purified fluorine-containing ether compound.

In the composition containing a purified fluorine-containing ether compound obtained through step (B), the purity of the fluorine-containing ether compound represented by general formula (1) is normally 95% by mass or more, preferably 97% by mass or more, more preferably 99% by mass or more, even more preferably 99.3% by mass or more, and particularly preferably 99.5% by mass or more.

In the composition containing a purified fluorine-containing ether compound obtained through step (B), the purity of the fluorine-containing olefin compound represented by general formula (2) is normally 0.1% by mass or less, preferably 0.05% by mass or less, more preferably 0.01% by mass or less, even more preferably 0.005% by mass or less, and particularly preferably 0.001% by mass or less.

The separation operation is preferably distillation, and examples thereof include simple distillation, extractive distillation, pressure swing distillation, and thin film distillation. Also, the separation operation is preferably rectification in which distillation is repeated. For the distillation (especially rectification), a distillation column with multiple theoretical stages (especially rectification column) can be used, and either continuous distillation or batch distillation may be employed. The pressure at which the distillation (especially rectification) is performed is preferably -0.05 MPaG or more, and preferably 0.10 MPaG or less. In the case where the distillation (especially rectification) is performed within such a pressure range, the boiling point difference among the fluorine-containing ether compound represented by general formula (1), the fluorine-containing olefin compound represented by general formula (2), and the adduct of the nitrogen-containing compound and/or the fluorine-containing carboxylic acid analogue can be made large (for example, the boiling point difference can be set to 10°C or more), which thus improves the separation operation and the separation accuracy.

The number of theoretical stages in the distillation column (especially rectification column) used in the distillation (especially rectification) is preferably 2 or more, and preferably 30 or less. The reflux ratio in the distillation column (especially rectification column) used in the distillation (especially rectification) is preferably 2 or more, and preferably 50 or less. The distillation column (especially rectification column) is preferably formed of a material that is resistant to corrosive action, such as glass, stainless steel (SUS), Hastelloy, Inconel, and Monel, and more preferably formed of SUS. Examples of the filler used in the distillation column (especially rectification column) include Raschig ring and Macmahon packing.

According to the production method of the present disclosure including steps (A) and (B) described above, a composition can be obtained that contains a purified fluorine-containing ether compound represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time.

### 2. Composition

The composition of the present disclosure encompasses two types: a composition obtained through step (A) in the production method of the present disclosure (pre-purification composition) and a composition obtained through step (A) and step (B) (post-purification composition). Hereinafter, each will be described.

### Pre-Purification Composition

The pre-purification composition of the present disclosure is a composition containing a fluorine-containing ether compound, wherein
the fluorine-containing ether compound is represented by general formula (1): CHX¹X²CF₂OX^{3,} wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
the total content of the adduct of the nitrogen-containing compound and/or the fluorine-containing carboxylic acid analogue is 5% by mass or less with respect to the mass of the fluorine-containing ether compound (100% by mass). Note that either the adduct of the nitrogen-containing compound or the fluorine-containing carboxylic acid analogue may be contained, or both may be contained, including the case where the content of one is substantially 0% by mass.

The total content of the adduct of the nitrogen-containing compound and/or the fluorine-containing carboxylic acid analogue is preferably 4.5% by mass or less, more preferably 4.0% by mass or less, with respect to the mass of the entire composition (100% by mass).

Here, when individually specifying the content of each of the adduct of the nitrogen-containing compound and/or the fluorine-containing carboxylic acid analogue, the pre-purification composition of the present disclosure is a composition containing a fluorine-containing ether compound, wherein
the fluorine-containing ether compound is represented by general formula (1): CHX¹X²CF₂OX^{3,} wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
the content of the adduct of the nitrogen-containing compound is 3% by mass or less with respect to the mass of the fluorine-containing ether compound (100% by mass), and the content of the fluorine-containing carboxylic acid analogue is 2% by mass or less with respect to the mass of the fluorine-containing ether compound (100% by mass). Note that either the adduct of the nitrogen-containing compound or the fluorine-containing carboxylic acid analogue may be contained, or both may be contained, including the case where the content of one is substantially 0% by mass.

Also, in the pre-purification composition, in the case where the fluorine-containing ether compound represented by general formula (1) is 1,1,2,3,3,3-hexafluoropropyl methyl ether (HFE-356mec) and the fluorine-containing olefin compound represented by general formula (2) is CF₃CF=CFOCH₃ and/or CF₂=CFCF₂OCH₃,
the pre-purification composition of the present disclosure is a composition containing a fluorine-containing ether compound, wherein
the fluorine-containing ether compound is 1,1,2,3,3,3-hexafluoropropyl methyl ether (HFE-356mec),
the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
the total content of the adduct of the nitrogen-containing compound and/or the fluorine-containing carboxylic acid analogue is 5% by mass or less with respect to the mass of the fluorine-containing ether compound (100% by mass). Note that either the adduct of the nitrogen-containing compound or the fluorine-containing carboxylic acid analogue may be contained, or both may be contained, including the case where the content of one is substantially 0% by mass.

Here, when individually specifying the content of each of the adduct of the nitrogen-containing compound and/or the fluorine-containing carboxylic acid analogue, the pre-purification composition of the present disclosure is a composition containing a fluorine-containing ether compound, wherein
the fluorine-containing ether compound is 1,1,2,3,3,3-hexafluoropropyl methyl ether (HFE-356mec),
the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
the content of the adduct of the nitrogen-containing compound is 3% by mass or less with respect to the mass of the fluorine-containing ether compound (100% by mass), and the content of the fluorine-containing carboxylic acid analogue is 2% by mass or less with respect to the mass of the fluorine-containing olefin compound (100% by mass). Note that either the adduct of the nitrogen-containing compound or the fluorine-containing carboxylic acid analogue may be contained, or both may be contained, including the case where the content of one is substantially 0% by mass.

According to the production method of the present disclosure, in step (A) of converting, through a reaction between a fluorine-containing olefin compound represented by general formula (2) and a specific nitrogen-containing compound (nitrogen-containing compound A and/or nitrogen-containing compound B) in the presence of a fluorine-containing ether compound represented by general formula (1), the fluorine-containing olefin compound represented by general formula (2) into an adduct of the nitrogen-containing compound and/or a fluorine-containing carboxylic acid analogue, decomposition of the fluorine-containing ether compound represented by general formula (1) is suppressed. In the production method of the present disclosure, embodiments using nitrogen-containing compound B (tertiary amine compound) have superiority that the total content of the adduct of the nitrogen-containing compound and/or the fluorine-containing carboxylic acid analogue is 5% by mass or less with respect to the mass of the fluorine-containing ether compound (100% by mass) even in the state of pre-purification composition and that the yield of the target, purified fluorine-containing ether compound, is easy to be increased.

### Post-Purification Composition

The post-purification composition of the present disclosure is a composition containing a fluorine-containing ether compound, wherein
the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
the total content of the adduct of the nitrogen-containing compound and/or the fluorine-containing carboxylic acid analogue is 0.2% by mass or less with respect to the mass of the fluorine-containing ether compound (100% by mass). Note that either the adduct of the nitrogen-containing compound or the fluorine-containing carboxylic acid analogue may be contained, or both may be contained, including the case where the content of one is substantially 0% by mass.

As described above, when identified by the Mulliken charge density on a nitrogen atom, the nitrogen-containing compound that can be used in the present disclosure can be identified as a nitrogen-containing compound with a Mulliken charge density on a nitrogen atom of -0.65 or more and 0.1 or less, and therefore, the post-purification composition of the present disclosure can also be described as follows:
"A composition containing a fluorine-containing ether compound, wherein
the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound with a Mulliken charge density value on a nitrogen atom of -0.65 or more and 0.1 or less, the reaction product being an adduct of the nitrogen-containing compound and/or a fluorine-containing carboxylic acid analogue, and
the total content of the adduct of the nitrogen-containing compound and/or the fluorine-containing carboxylic acid analogue is 0.2% by mass or less with respect to the mass of the fluorine-containing ether compound.".

The total content of the adduct of the nitrogen-containing compound and/or the fluorine-containing carboxylic acid analogue is preferably 0.2% by mass or less, more preferably 0.1% by mass or less, with respect to the mass of the entire composition (100% by mass).

Here, when individually specifying the content of each of the adduct of the nitrogen-containing compound and/or the fluorine-containing carboxylic acid analogue, the pre-purification composition of the present disclosure is a composition containing a fluorine-containing ether compound, wherein
the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
the content of the adduct of the nitrogen-containing compound is 0.1% by mass or less with respect to the mass of the fluorine-containing ether compound (100% by mass), and the content of the fluorine-containing carboxylic acid analogue is 0.1% by mass or less with respect to the mass of the fluorine-containing ether compound (100% by mass). Note that either the adduct of the nitrogen-containing compound or the fluorine-containing carboxylic acid analogue may be contained, or both may be contained, including the case where the content of one is substantially 0% by mass.

Also, in the pre-purification composition described above, in the case where the fluorine-containing ether compound represented by general formula (1) is 1,1,2,3,3,3-hexafluoropropyl methyl ether (HFE-356mec) and the fluorine-containing olefin compound represented by general formula (2) is CF₃CF=CFOCH₃ and/or CF₂=CFCF₂OCH₃,
the pre-purification composition of the present disclosure is a composition containing a fluorine-containing ether compound, wherein
the fluorine-containing ether compound is 1,1,2,3,3,3-hexafluoropropyl methyl ether (HFE-356mec),
the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
the total content of the adduct of the nitrogen-containing compound and/or the fluorine-containing carboxylic acid analogue is 0.2% by mass or less with respect to the mass of the fluorine-containing ether compound (100% by mass). Note that either the adduct of the nitrogen-containing compound or the fluorine-containing carboxylic acid analogue may be contained, or both may be contained, including the case where the content of one is substantially 0% by mass.

Here, when individually specifying the content of each of the adduct of the nitrogen-containing compound and/or the fluorine-containing carboxylic acid analogue, the pre-purification composition of the present disclosure is a composition containing a fluorine-containing ether compound, wherein
the fluorine-containing ether compound is 1,1,2,3,3,3-hexafluoropropyl methyl ether (HFE-356mec),
the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
the content of the adduct of the nitrogen-containing compound is 0.1% by mass or less with respect to the mass of the fluorine-containing ether compound (100% by mass), and the content of the fluorine-containing carboxylic acid analogue is 0.1% by mass or less with respect to the mass of the fluorine-containing ether compound (100% by mass). Note that either the adduct of the nitrogen-containing compound or the fluorine-containing carboxylic acid analogue may be contained, or both may be contained, including the case where the content of one is substantially 0% by mass.

In a suitable embodiment, the post-purification composition of the present disclosure contains, as the purified fluorine-containing ether compound, HFE-356mec as an essential component, of which the purified fluorine-containing ether compound is preferably HFE-356mec (consists substantially solely of HFE-356mec). Note that, in addition to the purified fluorine-containing ether compound, the post-purification composition of the present disclosure does not contain or contains the adduct of the nitrogen-containing compound and/or the fluorine-containing carboxylic acid analogue. Here, not containing the adduct of the nitrogen-containing compound and/or the fluorine-containing carboxylic acid analogue means that they are below the detection limit value (N.D.) as determined by quantitative analysis by gas chromatography (GC). In this case, the post-purification composition of the present disclosure is composed substantially solely of HFE-356mec, which is highly useful in that HFE-356mec of higher purity can be obtained compared to conventional methods. In the case where the post-purification composition of the present disclosure contains the adduct of the nitrogen-containing compound and/or the fluorine-containing carboxylic acid analogue, the total content of the adduct of the nitrogen-containing compound and/or the fluorine-containing carboxylic acid analogue is preferably 0.1% by mass or less, more preferably 0.05% by mass or less, with respect to the mass of HFE-356mec (100% by mass), in terms of stability of the composition, stability of materials to be brought into contact with the composition, and other factors. Also, the total content of the adduct of the nitrogen-containing compound and/or the fluorine-containing carboxylic acid analogue is preferably 0.005% by mass or more, more preferably 0.01% by mass or more, with respect to the mass of HFE-356mec (100% by mass), in terms of stability of the composition, cost reduction at the time of HFE-356mec production, and other factors.

The post-purification composition of the present disclosure in a suitable embodiment may contain hydrogen fluoride. In the case where the post-purification composition of the present disclosure in a suitable embodiment contains hydrogen fluoride, the content of hydrogen fluoride is preferably 0.01% by mass or less, more preferably 0.001% by mass or less, with respect to the mass of HFE-356mec (100% by mass).

Although the application of the post-purification composition of the present disclosure is not limited, it can be suitably used as, for example, a solvent, a solvent for coating agents, a lubricant solvent, a solvent for fluorinated oils, a solvent for reactions, a solvent for extraction agents, a coating solution for organic EL, a heat medium, a refrigerant, a heat transfer fluid, a foaming agent, a fire extinguishing agent, a cleaning agent, a spinning solution, an electrolytic solution, an aerosol, a gas emulsion, or a solvent for electrophotographic devices.

### Examples

Examples and Comparative Examples are given below to more specifically describe embodiments of the present disclosure. However, the present disclosure is not limited to these Examples. Hereinafter, "room temperature" means 20 to 25°C.

### Example 1 (Nitrogen-containing compound is a chain amide compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 37 g of N,N-dimethylformamide, 100 g of water, and 17 g of potassium hydroxide were added, and the reaction was performed at normal pressure and 60°C for 5 hours. Note that, for N,N-dimethylformamide, the Mulliken charge was calculated by performing structural optimization calculation using quantum chemical calculation software Gaussian16 from Gaussian, Inc. by the density functional theory (DFT) with B3LYP as the functional and 6-31G+(d) as the basis function, and the Mulliken charge on a nitrogen atom was confirmed to be -0.110.

Next, GC and 19F-NMR analysis were performed to confirm that the nitrogen-containing compound adduct was 0.84% by mass and the fluorine-containing carboxylic acid analogue was 0.54% by mass. These contents are both proportions with respect to the mass of HFE-356mec (100% by mass) (hereinafter, the same applies to the pre-purification composition).

Thereafter, the fluorine ether layer and the aqueous layer were separated. The obtained fluorine ether layer was washed with water, and then rectified and purified to obtain the fluoroether with a purity of 99.9% with a yield of 93%. At this time, the adduct of the nitrogen-containing compound was N.D., and the fluorine-containing carboxylic acid analogue was N.D. These contents are both proportions with respect to the mass of HFE-356mec (100% by mass) (hereinafter, the same applies to the post-purification composition).

### Example 2 (Nitrogen-containing compound is a chain amide compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 20 g of N,N-dimethylacetamide, 100 g of water, and 17 g of potassium hydroxide were added, and the reaction was performed at normal pressure and 40°C for 5 hours. Note that, for N,N-dimethylacetamide, the Mulliken charge was calculated by performing structural optimization calculation by the DFT as in Example 1, and the Mulliken charge on a nitrogen atom was confirmed to be -0.217.

Next, GC and 19F-NMR analysis were performed to confirm that the nitrogen-containing compound adduct was 0.74% by mass and the fluorine-containing carboxylic acid analogue was 0.64% by mass.

Thereafter, the fluorine ether layer and the aqueous layer were separated. The obtained fluorine ether layer was washed with water, and then rectified and purified to obtain the fluoroether with a purity of 99.9% with a yield of 94%. At this time, the adduct of the nitrogen-containing compound was N.D., and the fluorine-containing carboxylic acid analogue was N.D.

### Example 3 (Nitrogen-containing compound is a chain amide compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 10 g of 3-methoxy-N,N-dimethylpropionamide, 50 g of water, and 17 g of sodium hydroxide were added, and the reaction was performed at normal pressure and room temperature for 24 hours. Note that, for 3-methoxy-N,N-dimethylpropionamide, the Mulliken charge was calculated by performing structural optimization calculation by the DFT as in Example 1, and the Mulliken charge on a nitrogen atom was confirmed to be -0.224.

Next, GC and 19F-NMR analysis were performed to confirm that the nitrogen-containing compound adduct was 0.70% by mass and the fluorine-containing carboxylic acid analogue was 0.68% by mass.

Thereafter, the fluorine ether layer and the aqueous layer were separated. The obtained fluorine ether layer was washed with water, and then rectified and purified to obtain the fluoroether with a purity of 99.9% with a yield of 95%. At this time, the adduct of the nitrogen-containing compound was N.D., and the fluorine-containing carboxylic acid analogue was N.D.

### Example 4 (Nitrogen-containing compound is a cyclic amide compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 25 g of N-methylpyrrolidone, 50 g of water, and 15 g of potassium carbonate were added, and the reaction was performed at normal pressure and 40°C for 24 hours. Note that, for N-methylpyrrolidone, the Mulliken charge was calculated by performing structural optimization calculation by the DFT as in Example 1, and the Mulliken charge on a nitrogen atom was confirmed to be -0.124.

Next, GC and 19F-NMR analysis were performed to confirm that the nitrogen-containing compound adduct was 0.80% by mass and the fluorine-containing carboxylic acid analogue was 0.58% by mass.

Thereafter, the fluorine ether layer and the aqueous layer were separated. The obtained fluorine ether layer was washed with water, and then rectified and purified to obtain the fluoroether with a purity of 99.9% with a yield of 91%. At this time, the adduct of the nitrogen-containing compound was N.D., and the fluorine-containing carboxylic acid analogue was N.D.

### Example 5 (Nitrogen-containing compound is a cyclic urethane compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 25 g of 2-azetidinone, 50 g of water, and 30 g of sodium carbonate were added, and the reaction was performed in liquid phase at normal pressure and 40°C for 24 hours. Note that, for 2-azetidinone, the Mulliken charge was calculated by performing structural optimization calculation by the DFT as in Example 1, and the Mulliken charge on a nitrogen atom was confirmed to be -0.517.

Next, GC and 19F-NMR analysis were performed to confirm that the nitrogen-containing compound adduct was 0.77% by mass and the fluorine-containing carboxylic acid analogue was 0.61% by mass.

Thereafter, the fluorine ether layer and the aqueous layer were separated. The obtained fluorine ether layer was washed with water and then rectified and purified to obtain the fluoroether with a purity of 99.9% with a yield of 91%. At this time, the adduct of the nitrogen-containing compound was N.D., and the fluorine-containing carboxylic acid analogue was N.D.

### Example 6 (Nitrogen-containing compound is a cyclic urethane compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 25 g of 2-pyrrolidone, 50 g of water, and 17 g of potassium hydroxide were added, and the reaction was performed in liquid phase at normal pressure and 40°C. Note that, for 2-pyrrolidone, the Mulliken charge was calculated by performing structural optimization calculation by the DFT as in Example 1, and the Mulliken charge on a nitrogen atom was confirmed to be - 0.496.

Next, GC and 19F-NMR analysis were performed to confirm that the nitrogen-containing compound adduct was 0.87% by mass and the fluorine-containing carboxylic acid analogue was 0.51% by mass.

Thereafter, the fluorine ether layer and the aqueous layer were separated. The obtained fluorine ether layer was washed with water, and then rectified and purified to obtain the fluoroether with a purity of 99.9% with a yield of 91%. At this time, the adduct of the nitrogen-containing compound was N.D., and the fluorine-containing carboxylic acid analogue was N.D.

### Example 7 (Nitrogen-containing compound is a chain urea compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 35 g of tetramethylurea, 50 g of water, and 17 g of potassium hydroxide were added, and the reaction was performed at normal pressure and 40°C for 24 hours. Note that, for tetramethylurea, the Mulliken charge was calculated by performing structural optimization calculation by the DFT as in Example 1, and the Mulliken charge on a nitrogen atom was confirmed to be - 0.256.

Next, GC and 19F-NMR analysis were performed to confirm that the nitrogen-containing compound adduct was 0.77% by mass and the fluorine-containing carboxylic acid analogue was 0.61% by mass.

Thereafter, the fluorine ether layer and the aqueous layer were separated. The obtained fluorine ether layer was washed with water, and then rectified and purified to obtain the fluoroether with a purity of 99.9% with a yield of 92%. At this time, the adduct of the nitrogen-containing compound was 0.03% by mass, and the fluorine-containing carboxylic acid analogue was 0.02% by mass.

### Example 8 (Nitrogen-containing compound is a cyclic urea compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 35 g of 1,3-dimethyl-2-imidazolidinone (DMI), 50 g of water, and 17 g of potassium hydroxide were added, and the reaction was performed at normal pressure and 60°C. Note that, for dimethylimidazolidinone, the Mulliken charge was calculated by performing structural optimization calculation by the DFT as in Example 1, and the Mulliken charge on a nitrogen atom was confirmed to be -0.232.

Next, GC and 19F-NMR analysis were performed to confirm that the nitrogen-containing compound adduct was 0.80% by mass and the fluorine-containing carboxylic acid analogue was 0.58% by mass.

Thereafter, the fluorine ether layer and the aqueous layer were separated. The obtained fluorine ether layer was washed with water, and then rectified and purified to obtain the fluoroether with a purity of 99.9% with a yield of 91%. At this time, the adduct of the nitrogen-containing compound was 0.03% by mass, and the fluorine-containing carboxylic acid analogue was 0.03% by mass.

### Example 9 (Nitrogen-containing compound is a chain urethane compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 4 g of ethyl N-methylcarbamate, 50 g of water, and 17 g of potassium hydroxide were added, and the reaction was performed at normal pressure and 60°C. Note that, for ethyl N-methylcarbamate, the Mulliken charge was calculated by performing structural optimization calculation by the DFT as in Example 1, and the Mulliken charge on a nitrogen atom was confirmed to be - 0.612.

Next, GC and 19F-NMR analysis were performed to confirm that the nitrogen-containing compound adduct was 0.75% by mass and the fluorine-containing carboxylic acid analogue was 0.63% by mass.

Thereafter, the fluorine ether layer and the aqueous layer were separated. The obtained fluorine ether layer was washed with water, and then rectified and purified to obtain the fluoroether with a purity of 99.9% with a yield of 91%. At this time, the adduct of the nitrogen-containing compound was N.D., and the fluorine-containing carboxylic acid analogue was N.D.

### Example 10 (Nitrogen-containing compound is a cyclic urethane compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 4 g of 3-methyl-2-oxazolidone, 250 g of water, and 50 g of potassium bicarbonate were added, and the reaction was performed at normal pressure and room temperature for 24 hours. Note that, for 3-methyl-2-oxazolidone, the Mulliken charge was calculated by performing structural optimization calculation by the DFT as in Example 1, and the Mulliken charge on a nitrogen atom was confirmed to be -0.257.

Next, GC and 19F-NMR analysis were performed to confirm that the nitrogen-containing compound adduct was 0.72% by mass and the fluorine-containing carboxylic acid analogue was 0.66% by mass.

Thereafter, the fluorine ether layer and the aqueous layer were separated. The obtained fluorine ether layer was washed with water, and then rectified and purified to obtain the fluoroether with a purity of 99.9% with a yield of 90%. At this time, the adduct of the nitrogen-containing compound was N.D., and the fluorine-containing carboxylic acid analogue was N.D.

### Example 11 (Nitrogen-containing compound is a chain imide compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 10 g of N-methyldiacetamide, 250 g of water, and 50 g of sodium bicarbonate were added, and the reaction was performed at normal pressure and room temperature for 24 hours. Note that, for N-methyldiacetamide, the Mulliken charge was calculated by performing structural optimization calculation by the DFT as in Example 1, and the Mulliken charge on a nitrogen atom was confirmed to be -0.327.

Next, GC and 19F-NMR analysis were performed to confirm that the nitrogen-containing compound adduct was 0.76% by mass and the fluorine-containing carboxylic acid analogue was 0.62% by mass.

Thereafter, the fluorine ether layer and the aqueous layer were separated. The obtained fluorine ether layer was washed with water and then rectified and purified to obtain the fluoroether with a purity of 99.9% with a yield of 90%. At this time, the adduct of the nitrogen-containing compound was N.D., and the fluorine-containing carboxylic acid analogue was N.D.

### Example 12 (Nitrogen-containing compound is a cyclic imide compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 15 g of N-methylsuccinimide, 50 g of water, and 17 g of potassium hydroxide were added, and the reaction was performed at normal pressure and 60°C for 24 hours. Note that, for N-methylsuccinimide, the Mulliken charge was calculated by performing structural optimization calculation by the DFT as in Example 1, and the Mulliken charge on a nitrogen atom was confirmed to be -0.197.

Next, GC and 19F-NMR analysis were performed to confirm that the nitrogen-containing compound adduct was 0.80% by mass and the fluorine-containing carboxylic acid analogue was 0.48% by mass.

Thereafter, the fluorine ether layer and the aqueous layer were separated. The obtained fluorine ether layer was washed with water, and then rectified and purified to obtain the fluoroether with a purity of 99.9% with a yield of 91%. At this time, the adduct of the nitrogen-containing compound was 0.05% by mass, and the fluorine-containing carboxylic acid analogue was N.D.

### Example 13 (Nitrogen-containing compound is a chain amide compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 1 g of N,N-dimethylformamide, 50 g of water, and 17 g of potassium hydroxide were added, and the reaction was performed at normal pressure and 60°C for 24 hours. Note that, for N,N-dimethylformamide, the Mulliken charge was calculated by performing structural optimization calculation by the DFT as in Example 1, and the Mulliken charge on a nitrogen atom was confirmed to be -0.347.

Next, GC and 19F-NMR analysis were performed to confirm that the adduct of the nitrogen-containing compound was 0.62% by mass and the fluorine-containing carboxylic acid analogue was 0.76% by mass.

Thereafter, the fluorine ether layer and the aqueous layer were separated. The obtained fluorine ether layer was washed with water, and then rectified and purified to obtain the fluoroether with a purity of 99.9% with a yield of 91%. At this time, the adduct of the nitrogen-containing compound was N.D., and the fluorine-containing carboxylic acid analogue was N.D.

### Example 14 (Nitrogen-containing compound is a chain amine of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 6 g of trimethylamine, 50 g of water, and 17 g of potassium hydroxide were added, and the reaction was performed at normal pressure and 60°C for 24 hours. Note that, for trimethylamine, the Mulliken charge was calculated by performing structural optimization calculation by the DFT as in Example 1, and the Mulliken charge on a nitrogen atom was confirmed to be - 0.168.

Next, GC and 19F-NMR analysis were performed to confirm that the nitrogen-containing compound adduct was N.D. and the fluorine-containing carboxylic acid analogue was 1.25% by mass.

Thereafter, the fluorine ether layer and the aqueous layer were separated. The obtained fluorine ether layer was washed with water, and then rectified and purified to obtain the fluoroether with a purity of 99.9% with a yield of 86%. At this time, the adduct of the nitrogen-containing compound was N.D., and the fluorine-containing carboxylic acid analogue was N.D.

### Example 15 (Nitrogen-containing compound is a chain amine of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 10 g of triethylamine, 50 g of water, and 17 g of potassium hydroxide were added, and the reaction was performed at normal pressure and 60°C for 24 hours. Note that, for triethylamine, the Mulliken charge was calculated by performing structural optimization calculation by the DFT as in Example 1, and the Mulliken charge on a nitrogen atom was confirmed to be 0.041.

Next, GC and 19F-NMR analysis were performed to confirm that the nitrogen-containing compound adduct was N.D. and the fluorine-containing carboxylic acid analogue was 1.17% by mass.

Thereafter, the fluorine ether layer and the aqueous layer were separated. The obtained fluorine ether layer was washed with water and then rectified and purified to obtain the fluoroether with a purity of 99.9% with a yield of 86%. At this time, the adduct of the nitrogen-containing compound was N.D., and the fluorine-containing carboxylic acid analogue was N.D.

### Example 16 (Nitrogen-containing compound is a cyclic amine of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 15 g of diazabicycloundecene (hereinafter, DBU), 50 g of water, and 17 g of potassium hydroxide were added, and the reaction was performed at normal pressure and 60°C for 24 hours. Note that, for DBU, the Mulliken charge was calculated by performing structural optimization calculation by the DFT as in Example 1, and the Mulliken charge on a nitrogen atom was confirmed to be -0.255.

Next, GC and 19F-NMR analysis were performed to confirm that the nitrogen-containing compound adduct was N.D. and the fluorine-containing carboxylic acid analogue was 0.84% by mass.

Thereafter, the fluorine ether layer and the aqueous layer were separated. The obtained fluorine ether layer was washed with water, and then rectified and purified to obtain the fluoroether with a purity of 99.9% with a yield of 90%. At this time, the adduct of the nitrogen-containing compound was N.D., and the fluorine-containing carboxylic acid analogue was N.D.

### Example 17 (Nitrogen-containing compound is a cyclic amine of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 9 g of N-methylpyrrolidine, 50 g of water, and 17 g of potassium hydroxide were added, and the reaction was performed at normal pressure and 60°C for 24 hours. Note that, for N-methylpyrrolidine, the Mulliken charge was calculated by performing structural optimization calculation by the DFT as in Example 1, and the Mulliken charge on a nitrogen atom was confirmed to be -0.127.

Next, GC and 19F-NMR analysis were performed to confirm that the nitrogen-containing compound adduct was N.D. and the fluorine-containing carboxylic acid analogue was 0.99% by mass.

Thereafter, the fluorine ether layer and the aqueous layer were separated. The obtained fluorine ether layer was washed with water, and then rectified and purified to obtain the fluoroether with a purity of 99.9% with a yield of 87%. At this time, the adduct of the nitrogen-containing compound was N.D., and the fluorine-containing carboxylic acid analogue was N.D.

### Example 18 (Nitrogen-containing compound is a heterocyclic aromatic compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 8 g of pyridine, 50 g of water, and 17 g of potassium hydroxide were added, and the reaction was performed at normal pressure and 60°C for 24 hours. Note that, for pyridine, the Mulliken charge was calculated by performing structural optimization calculation by the DFT as in Example 1, and the Mulliken charge on a nitrogen atom was confirmed to be -0.179.

Next, GC and 19F-NMR analysis were performed to confirm that the nitrogen-containing compound adduct was N.D. and the fluorine-containing carboxylic acid analogue was 1.05% by mass.

Thereafter, the fluorine ether layer and the aqueous layer were separated. The obtained fluorine ether layer was washed with water, and then rectified and purified to obtain the fluoroether with a purity of 99.9% with a yield of 86%. At this time, the adduct of the nitrogen-containing compound was N.D., and the fluorine-containing carboxylic acid analogue was N.D.

### Example 19 (Nitrogen-containing compound is a heterocyclic aromatic compound of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 12 g of 4-dimethylaminopyridine, 50 g of water, and 17 g of potassium hydroxide were added, and the reaction was performed at normal pressure and 60°C for 24 hours. Note that, for 4-dimethylaminopyridine, the Mulliken charge was calculated by performing structural optimization calculation by the DFT as in Example 1, and the Mulliken charge on a nitrogen atom was confirmed to be -0.210.

Next, GC and 19F-NMR analysis were performed to confirm that the nitrogen-containing compound adduct was N.D. and the fluorine-containing carboxylic acid analogue was 0.89% by mass.

Thereafter, the fluorine ether layer and the aqueous layer were separated. The obtained fluorine ether layer was washed with water, and then rectified and purified to obtain the fluoroether with a purity of 99.9% with a yield of 89%. At this time, the adduct of the nitrogen-containing compound was N.D., and the fluorine-containing carboxylic acid analogue was N.D.

### Example 20 (Nitrogen-containing compound is a cyclic amine of the present disclosure)

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass], and hydrogen fluoride with a purity of 0.035% by mass were charged.

Next, 14 g of N,N-dimethylbenzylamine, 50 g of water, and 17 g of potassium hydroxide were added, and the reaction was performed at normal pressure and 60°C for 24 hours. Note that, for dimethylbenzylamine, the Mulliken charge was calculated by performing structural optimization calculation by the DFT as in Example 1, and the Mulliken charge on a nitrogen atom was confirmed to be -0.100.

Next, GC and 19F-NMR analysis were performed to confirm that the nitrogen-containing compound adduct was N.D. and the fluorine-containing carboxylic acid analogue was 0.95% by mass.

Thereafter, the fluorine ether layer and the aqueous layer were separated. The obtained fluorine ether layer was washed with water, and then rectified and purified to obtain the fluoroether with a purity of 99.9% with a yield of 88%. At this time, the adduct of the nitrogen-containing compound was N.D., and the fluorine-containing carboxylic acid analogue was N.D.

### Comparative Example 1 (Nitrogen-containing compound is amylamine (primary amine and comparative product))

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass] and hydrogen fluoride with a purity of 0.035% by mass were charged.

Next, 25 g of amylamine, 25 g of water, and 17 g of potassium hydroxide were added, and the reaction was performed at normal pressure and 40°C for 24 hours. Note that, for amylamine, the Mulliken charge was calculated by performing structural optimization calculation by the DFT as in Example 1, and the Mulliken charge on a nitrogen atom was confirmed to be -0.821.

Thereafter, the fluorine ether layer and the aqueous layer were separated. The obtained fluorine ether layer was washed with water, and then rectified and purified to obtain the fluoroether with a purity of 99.8% with a yield of 79%.

### Comparative Example 2 (Nitrogen-containing compound is benzylamine (primary amine and comparative product))

In a 1 L glass container, 182 g of a crude product containing HFE-356mec with a purity of 94.4% by mass, a fluorine-containing olefin compound with a purity of 1.38% by mass [CF₃CF=CFOCH₃ (olefin 1) with a purity of 1.28% by mass and CF₂=CFCF₂OCH₃ (olefin 2 (E,Z)) with a purity of 0.10% by mass] and hydrogen fluoride with a purity of 0.035% by mass were charged.

Next, 25 g of benzylamine, 25 g of water, and 17 g of potassium hydroxide were added, and the reaction was performed at normal pressure and 40°C for 24 hours. Note that, for benzylamine, the Mulliken charge was calculated by performing structural optimization calculation by the DFT as in Example 1, and the Mulliken charge on a nitrogen atom was confirmed to be -0.814.

Thereafter, the fluorine ether layer and the aqueous layer were separated. The obtained fluorine ether layer was washed with water, and then rectified and purified to obtain the fluoroether with a purity of 99.8% with a yield of 80%.

## Claims

1. A method for producing a composition containing a purified fluorine-containing ether compound, wherein
the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time, and
the method comprises:
(A) converting, through a reaction between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound in the presence of the fluorine-containing ether compound represented by general formula (1), the fluorine-containing olefin compound represented by general formula (2) into an adduct of the nitrogen-containing compound and/or a fluorine-containing carboxylic acid analogue to obtain a composition containing the fluorine-containing ether compound represented by general formula (1) and the adduct of the nitrogen-containing compound and/or the fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound; and
(B) separating the adduct of the nitrogen-containing compound and/or the fluorine-containing carboxylic acid analogue from the composition obtained in step (A) to obtain the composition containing a purified fluorine-containing ether compound.

2. The production method according to claim 1, wherein the nitrogen-containing compound is at least one selected from the group consisting of an amide compound, a urea compound, a urethane compound, an imide compound, and a tertiary amine compound.

3. The production method according to claim 1 or 2, wherein in step (B), the adduct of the nitrogen-containing compound and/or the fluorine-containing carboxylic acid analogue are separated from the composition obtained in step (A) by distillation of the composition obtained in step (A).

4. The production method according to any one of claims 1 to 3, wherein the fluorine-containing ether compound represented by general formula (1) is 1,1,2,3,3,3-hexafluoropropyl methyl ether (HFE-356mec).

5. The production method according to any one of claims 1 to 4, wherein the fluorine-containing olefin compound represented by general formula (2) is CF₃CF=CFOCH₃ and/or CF₂=CFCF₂OCH₃.

6. A composition containing a fluorine-containing ether compound, wherein
the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
a total content of the adduct of the nitrogen-containing compound and/or the fluorine-containing carboxylic acid analogue is 5% by mass or less with respect to a mass of the fluorine-containing ether compound.

7. A composition containing a fluorine-containing ether compound, wherein
the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
a content of the adduct of the nitrogen-containing compound is 3% by mass or less with respect to a mass of the fluorine-containing ether compound, and a content of the fluorine-containing carboxylic acid analogue is 2% by mass or less with respect to a mass of the fluorine-containing ether compound.

8. A composition containing a fluorine-containing ether compound, wherein
the fluorine-containing ether compound is 1,1,2,3,3,3-hexafluoropropyl methyl ether (HFE-356mec),
the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
a total content of the adduct of the nitrogen-containing compound and/or the fluorine-containing carboxylic acid analogue is 5% by mass or less with respect to a mass of the fluorine-containing ether compound.

9. A composition containing a fluorine-containing ether compound, wherein
the fluorine-containing ether compound is 1,1,2,3,3,3-hexafluoropropyl methyl ether (HFE-356mec),
the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
a content of the adduct of the nitrogen-containing compound is 3% by mass or less with respect to a mass of the fluorine-containing ether compound, and a content of the fluorine-containing carboxylic acid analogue is 2% by mass or less with respect to a mass of the fluorine-containing olefin compound.

10. A composition containing a fluorine-containing ether compound, wherein
the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
a total content of the adduct of the nitrogen-containing compound and/or the fluorine-containing carboxylic acid analogue is 0.2% by mass or less with respect to a mass of the fluorine-containing ether compound.

11. A composition containing a fluorine-containing ether compound, wherein
the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
a content of the adduct of the nitrogen-containing compound is 0.1% by mass or less with respect to a mass of the fluorine-containing ether compound, and a content of the fluorine-containing carboxylic acid analogue is 0.1% by mass or less with respect to a mass of the fluorine-containing ether compound.

12. A composition containing a fluorine-containing ether compound, wherein
the fluorine-containing ether compound is 1,1,2,3,3,3-hexafluoropropyl methyl ether (HFE-356mec),
the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
a total content of the adduct of the nitrogen-containing compound and/or the fluorine-containing carboxylic acid analogue is 0.2% by mass or less with respect to a mass of the fluorine-containing ether compound.

13. A composition containing a fluorine-containing ether compound, wherein
the fluorine-containing ether compound is 1,1,2,3,3,3-hexafluoropropyl methyl ether (HFE-356mec),
the composition contains a reaction product between a fluorine-containing olefin compound represented by CF₃CF=CFOCH₃ and/or CF₂=CFCF₂OCH₃, and a nitrogen-containing compound, the reaction product being an adduct of the nitrogen-containing compound and/or a fluorine-containing carboxylic acid analogue, wherein the nitrogen-containing compound is nitrogen-containing compound A having a structure in which an acyl group is bonded to a nitrogen atom and/or nitrogen-containing compound B that is a tertiary amine compound, and
a content of the adduct of the nitrogen-containing compound is 0.1% by mass or less with respect to a mass of the fluorine-containing ether compound, and a content of the fluorine-containing carboxylic acid analogue is 0.1% by mass or less with respect to a mass of the fluorine-containing ether compound.

14. A method for producing a composition containing a purified fluorine-containing ether compound, wherein
the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time, and
the method comprises:
(A) converting, through a reaction between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound with a Mulliken charge density value on a nitrogen atom of -0.65 or more and 0.1 or less in the presence of the fluorine-containing ether compound represented by general formula (1), the fluorine-containing olefin compound represented by general formula (2) into an adduct of the nitrogen-containing compound and/or a fluorine-containing carboxylic acid analogue to obtain a composition containing the fluorine-containing ether compound represented by general formula (1) and the adduct of the nitrogen-containing compound and/or the fluorine-containing carboxylic acid analogue; and
(B) separating the adduct of the nitrogen-containing compound and/or the fluorine-containing carboxylic acid analogue from the composition obtained in step (A) to obtain the composition containing a purified fluorine-containing ether compound.

15. A composition containing a fluorine-containing ether compound, wherein
the fluorine-containing ether compound is represented by general formula (1): CHX¹X²CF₂OX³, wherein X¹ and X² are the same or different and represent a hydrogen atom, a fluorine atom, or a trifluoromethyl group, and X³ represents an alkyl group having 1 to 6 carbon atoms and optionally containing a fluorine atom, provided that X¹ and X² are not trifluoromethyl groups at the same time,
the composition contains a reaction product between a fluorine-containing olefin compound represented by general formula (2): CX¹X²=CF(CF₂)ₙOX³, wherein n represents 0 or 1, and X¹, X² and X³ are the same as described above, provided that when X¹ or X² is a trifluoromethyl group, n is 0, and X¹ and X² are not trifluoromethyl groups at the same time, and a nitrogen-containing compound with a Mulliken charge density value on a nitrogen atom of -0.65 or more and 0.1 or less, the reaction product being an adduct of the nitrogen-containing compound and/or a fluorine-containing carboxylic acid analogue, and
a total content of the adduct of the nitrogen-containing compound and/or the fluorine-containing carboxylic acid analogue is 0.2% by mass or less with respect to a mass of the fluorine-containing ether compound.
